# EUROPÄISCHE PATENTSCHRIFT

(11) **EP 3 250 133 B1**
(45) Veröffentlichungstag und Bekanntmachung des Hinweises auf die Patenterteilung: **07.10.2020**
(21) Anmeldenummer: 15817172.8
(22) Anmeldetag: 15.12.2015
(51) Int. Cl.: A61B 17/16, A61B 17/29

(54) **SCHAFTINSTRUMENT FÜR CHIRURGISCHE ZWECKE**
SHAFT INSTRUMENT FOR SURGICAL PURPOSES
INSTRUMENT D'ARBRE POUR BUTS CHIRURGICALS

(30) Priorität: 26.01.2015 EP 15152482
(43) Veröffentlichungstag der Anmeldung: 06.12.2017
(73) Patentinhaber: Qenus Medical AG, 9050 Appenzell (CH)
(72) Erfinder: KOLLER, Martin, 9050 Appenzell (CH); KÜLLING, Fabrice, 9200 Gossau (CH)
(74) Vertreter: Hepp Wenger Ryffel AG
(86) Internationale Anmeldenummer: PCT/EP2015/079822
(87) Internationale Veröffentlichungsnummer: WO 2016/119972

(56) Entgegenhaltungen:
- EP-A1- 0 621 084
- WO-A1-89/01322
- US-A- 5 961 531
- US-B1- 6 214 010

## Beschreibung

Die Erfindung betrifft ein Schaftinstrument für chirurgische Zwecke gemäss dem Oberbegriff von Anspruch 1. Derartige Instrumente dienen unter anderem dazu, an teilweise schwer zugänglichen Körperstellen Resektionen vorzunehmen oder Gewebeproben zu entnehmen, wie beispielsweise bei einer Laminektomie.

Gattungsmässig vergleichbare Instrumente sind in der Chirurgie seit längerer Zeit bekannt und gebräuchlich. So beschreibt die EP 1 491 155 A1 eine Knochen- und Gewebestanze, welche als Schiebeschaftinstrument ausgeführt ist. Das besagte Instrument weist einen länglichen Schaft auf, der an einem Ende fest mit einem Schaftgriff verbunden ist. Auf dem Schaft ist ein Schieber in Längsrichtung verschiebbar gelagert. Der Schieber steht mit einem unter Federvorspannung stehenden Schiebergriff in Wirkverbindung und kann durch Betätigen des Schiebergriffs verschoben werden. Die hierzu benötigte und von der Hand des Chirurgen ausgeübte Kraft wird dabei vom Schiebergriff über eine Rotationsbewegung aufgenommen.

Ein Nachteil der bekannten Schaftinstrumente besteht darin, dass es bei deren Betätigung zu einem leichten Verkippen des Instrumentes in der Hand des Operateurs kommt. Dadurch ist insbesondere ein präzises Führen der Schaftspitze während der Betätigung des Instrumentes erschwert. Es ist daher eine Aufgabe der Erfindung, die Nachteile im Stand der Technik zu überwinden.

Insbesondere ist es eine Aufgabe der Erfindung, ein vielfältig anwendbares und konstruktiv einfaches Schaftinstrument der eingangs erwähnten Art zu schaffen, welches mit einem hohen Grad an Präzision und Sicherheit geführt und betätigt werden kann. Darüber hinaus soll das Instrument eine kompakte Bauweise und eine hohe Robustheit aufweisen. Ferner soll es effizient im klinischen Umfeld handhabbar, insbesondere leicht sterilisierbar sein. Ausserdem soll das Schaftinstrument möglichst langlebig und zu einem günstigen Preis fertigbar sein.

Diese Aufgaben werden durch ein Schaftinstrument gelöst, welches die Merkmale im Anspruch 1 aufweist.

Die Erfindung betrifft ein Schaftinstrument für chirurgische Zwecke mit einem Schaftstück, das an einem Ende fest mit einem Schaftgriff verbunden ist. Ferner weist das Schaftinstrument ein Gleitstück auf, welches relativ zum Schaftstück in dessen Längsrichtung verschiebbar gelagert ist. An einem Ende steht das Gleitstück derart mit einem unter Vorspannung stehenden Handgriff in Wirkverbindung, dass durch Betätigen des Handgriffs das Gleitstück gegen die Vorspannung verschiebbar : ist, wobei das Gleitstück als parallel und neben dem Schaftstück angeordneter Schieber ausgebildet ist. Die Erfindung zeichnet sich dadurch aus, dass an wenigstens zwei voneinander beabstandeten Schaftgelenken am Schaftstück oder am Schaftgriff jeweils ein Hebelarm angelenkt ist. Ferner weist der Handgriff einen Koppelabschnitt auf, welcher die wenigstens zwei Hebelarme an voneinander beabstandeten Koppelgelenken gleichsinnig schwenkbar miteinander verbindet, sowie einen Griffabschnitt, welcher sich quer zum Koppelabschnitt und zum Schaftstück erstreckt.

Durch die Aufhängung des Handgriffs an mehreren Hebelarmen entspricht die Kraftübertragung von der Hand auf den Griffabschnitt mehr einer Translation denn einer Rotation. Dies ermöglicht eine bessere Kraftübertragung auf das Instrument, vor allem aber eine ruhigere Führung der Schaftspitze.

Die Kraftübertragung wird insbesondere dadurch verbessert, dass jeder auf den Handgriff wirkende Finger seine Kraft über die Koppelgelenke auf die Hebelarme überträgt. Damit wird eine grössere Hebelwirkung erzielt.

Es versteht sich von selbst, dass die vorliegende Griffkonstruktion bei Schaftinstrumenten aller Art, insbesondere bei Schiebeschaft- und Rohrschaftinstrumenten, anwendbar ist. So kann der besagte Aufbau nicht nur bei Knochen und Gewebestanzen, sondern auch bei Scheren- oder Griffinstrumenten eingesetzt werden.

Die Koppelgelenke können an den dem Schaftstück oder Schaftgriff gegenüberliegenden Enden der Hebelarme liegen. Durch diese Anlenkung der Hebelarme durch den Handgriff wird bei einem erfindungsgemässen Schaftinstrument eine maximale Hebelwirkung erzielt.

Allerdings ist es auch möglich, dass die Koppelgelenke in einem mittleren Bereich von zumindest einem Hebelarm angeordnet sind. Dies ermöglicht das Anbringen weiterer Elemente, wie z.B. Arretierungsmitteln, an den Hebelarmen.

Zumindest ein Hebelarm kann als Doppelhebelarm mit einem Kraftarm und einem Lastarm ausgebildet sein, wobei der Kraftarm mit dem Koppelabschnitt des Handgriffes und der Lastarm mit dem Gleitstück in Wirkverbindung stehen. Dieser Aufbau ermöglicht eine besonders wirkungsvolle mechanische Kopplung des Hebelarms mit dem Gleitstück bei einer kompakten Bauweise des Schaftinstrumentes.

Die Schaftgelenke können in Querrichtung zum Schaftstück versetzt angeordnet sein. Eine versetzte Anordnung der Schaftgelenke am Schaftstück oder am Schaftgriff ist insbesondere bei der Ausbildung eines Hebelarms als Doppelhebelarm vorteilhaft, da dadurch ein weitaus kompakterer Aufbau des Instrumentes ermöglicht wird.

Das Verhältnis der Länge des längsten Hebelarmes zum zum längsten Abstand zwischen zwei Koppelgelenken kann in Ruhestellung in einem Bereich von 2 bis 20, vorzugsweise von 3 bis 10, bevorzugterweise von 4 bis 6 liegen. Durch diese Proportionierung der Griffskonstruktion wird eine möglichst optimale Kraftübertragung erzielt.

An zumindest einem der Hebelarme kann ein Anschlagselement angebracht sein, welches die Winkelstellung des Hebelarms in Ruhestellung definiert. Dieses Anschlagselement kann stufenlos einstellbar ausgeführt sein. Dadurch ist es dem Operateur möglich, vor Verwendung des Schaftinstrumentes dessen Ruhestellung einzustellen und damit auf den bevorstehenden Eingriff anzupassen.

Das Gleitstück ist als parallel und neben dem Schaftstück angeordneter Schieber ausgebildet. Derartige Schaftinstrumente werden auch als Schiebeschaftinstrumente bezeichnet. Ein Schiebeschaftinstrument hat den Vorteil, dass zu dessen Reinigung, insbesondere Sterilisation vor einem chirurgischen Eingriff, der Schieber leicht vom Schaftstück getrennt werden kann. Dadurch sind auch die innenliegenden Flächen leicht zugänglich. So kann der Schieber derart am Schaftstück angelenkt sein, dass er über eine Klappstellung vom Schaftstück freistellbar ist. Dies ermöglicht eine Reinigung des Instrumentes, ohne dass Schieber und Schaftstück getrennt werden müssen. Dies vereinfacht die Handhabung im klinischen Alltag und verhindert, dass zwei Elemente unterschiedlicher Einheiten nach dem Reinigungsvorgang zusammengefügt werden.

In einer nicht erfindungsgemässen Ausführungsform können das Schaftstück und das Gleitstück auch als zwei im Wesentlichen koaxial angeordnete Elemente ausgebildet sein, wobei das Schaftstück eine, vorzugsweise röhrenförmige, Aufnahme für das Gleitstück bildet. Dieser Aufbau eines Schaftinstrumentes wird auch als Rohrschaftinstrument bezeichnet. Rohrschaftinstrumente haben den Vorteil, dass sie keine relativ zueinander verschiebbaren Teile an deren Aussenfläche aufweisen. Dadurch wird ihre Betätigung in besonders engen Zugangskanälen zu einem Operationsbereich besonders erleichtert.

Das Verhältnis der Länge des Schaftes zur Länge des längsten Hebelarms kann in einem Bereich von 1 bis 10, vorzugsweise von 1.2 bis 4, bevorzugterweise von 1.5 bis 2.8 liegen. Ein derartiges Schaftinstrument weist eine besonders vorteilhafte Ergonomie auf und lässt sich leicht in der Hand eines Chirurgen führen. Ausserdem ist bei diesem Verhältnis noch eine tolerierbare Ablenkung der Schaftspitze bei voller Betätigung des Handgriffs gewährleistet.

Das Schaftstück und das Gleitstück können, vorzugsweise über einen reversiblen Formschluss, insbesondere über einen Schwalbenschwanz mit einem Sicherungsstift, lösbar mit dem Schaftgriff verbunden sein. Dadurch, dass das Schaftstück und das Gleitstück vom Schaftgriff trennbar sind, lassen sich verschiedene Schaftkonfigurationen an ein und denselben Schaftgriff anbringen. So ist es denkbar, ein System von Schaftinstrumenten bereitzustellen, bei dem verschiedene Schaftkonfigurationen mit unterschiedlichen Schaftlängen oder verschiedenen Werkzeugen an einem Schaftgriff mit identischer Bauweise angebracht werden. Durch diese modulare Bauweise lässt sich eine Vielzahl verschiedener Schaftinstrumente für unterschiedliche Anwendungsbereiche leicht bereitstellen.

Die Vorspannung kann durch ein am Schaftgriff angebrachtes und auf einen der Hebelarme wirkendes Federelement, insbesondere durch eine Blattfeder erzeugt werden. Dadurch wird auf zuverlässige Weise und mit einer besonders einfachen Konstruktion erreicht, dass der Handgriff nach Betätigung des Schaftinstrumentes wieder seine Ruhestellung einnimmt. Hierzu kann einer der Hebelarme eine Verankerungsstruktur für das Federelement, insbesondere eine Führungsnut für eine Blattfeder, aufweisen. Ferner kann das Federelement einen in der Verankerungsstruktur verankerten Gleitkörper insbesondere eine an einer Blattfeder angebrachte Kugel, aufweisen. Dies ermöglicht eine zuverlässige Führung des Federelementes.

Das Schaftstück kann ein Führungsprofil und der Schieber ein darin eingreifendes Eingriffselement aufweisen. Allerdings ist es auch möglich, dass der Schieber ein Führungsprofil und das Schaftstück ein darin eingreifendes Eingriffselement ausweist. Die Kombination von Führungsprofil und Eingriffselement ermöglicht es, den Schieber besonders stabil und in Längsrichtung verschiebbar am Schaft zu lagern. Es versteht sich von selbst, dass Schieber und Schaftstück auch mehrere Führungsprofile bzw. Eingriffselemente aufweisen können. Das Führungsprofil und das Eingriffselement können jeweils einstückig mit dem Schaft bzw. dem Schieber ausgeführt sein. Ein derartiges Schaftinstrument kommt mit wenigen Einzelteilen aus und ist einfach zu reinigen bzw. zu sterilisieren.

Das Anschlagselement kann die Winkelstellung des Hebelarms in Ruhestellung in einer sog. Verriegelungsposition definieren. Zusätzlich kann es in eine sog. Entriegelungsposition bewegbar, insbesondere verschiebbar oder schwenkbar, sein. Dabei kann mit dem Anschlagselement in Entriegelungsposition der Hebelarm entgegen der Betätigungsrichtung über die Ruhestellung hinaus bewegbar und der Schieber dadurch zumindest teilweise vom Schaftstück lösbar sein. Bei dieser Ausführung kann der Schieber durch ausfahren von zumindest einem Eingriffselement aus einem Führungsprofil entgegen der Betätigungsrichtung vom Schaftstück lösbar sein. Dies stellt eine besonders einfache Lösung dar, um das Schaftinstrument in seinem montierten Zustand zuverlässig zu sichern und es nach entsichern leicht zu demontieren.

Der Lastarm kann am dem Kraftarm gegenüberliegenden Ende ein Gabelprofil aufweisen, in das ein am Gleitstück angebrachter Bolzen einsetzbar ist. Dies ermöglicht eine besonders zuverlässige Kraftübertragung vom Kraftarm auf das Gleitstück, wobei die Zerlegung des Schaftinstrumentes dennoch leicht zu bewerkstelligen ist, da der Bolzen einfach aus dem Gabelprofil ausgefahren werden kann.

Dabei kann das Gabelprofil asymmetrisch ausgestaltet sein, wobei die bei Betätigung des Schaftinstrumentes von dem am Gleitstück angebrachten Bolzen beaufschlagte Seite des Gabelprofils eine höhere Materialstärke aufweist. Experimente haben gezeigt, dass bei symmetrischem Gabelprofil die Gefahr besteht, dass dieses unter den bei der Verwendung des Schaftinstrumentes auftretenden Kräften deformiert wird. Durch die höhere Materialstärke des Gabelprofils auf der Seite, die einer stärkeren Belastung ausgesetzt ist, kann dem vorgebeugt werden.

Weitere Vorteile und Einzelmerkmale der Erfindung ergeben sich aus der nachfolgenden Beschreibung eines Ausführungsbeispiels und aus den Zeichnungen.

Es zeigen schematisch:
- Figur 1:: Ein erfindungsgemässes Schaftinstrument in einer Seitenansicht in geöffnetem Zustand;
- Figur 2:: Das erfindungsgemässe Schaftinstrument gemäss Figur 1 in geschlossenem Zustand;
- Figur 3:: Prinzipiendarstellung der Griffkonstruktion eines erfindungsgemässen Schaftinstrumentes in geöffnetem Zustand in Seitenansicht;
- Figur 4:: Prinzipiendarstellung gemäss Figur 3 in geschlossenem Zustand;
- Figur 5:: Perspektivische Darstellung eines erfindungsgemässen Schaftinstrumentes gemäss der Figuren 1 und 2;
- Figur 6:: Perspektivische Darstellung des erfindungsgemässen Schaftinstrumentes gemäss Figur 5;
- Figur 7:: Darstellung des erfindungsgemässen Schaftinstrumentes gemäss Figur 6 in Seitenansicht in aufgeklapptem Zustand;
- Figur 8:: Perspektivische Darstellung eines weiteren Ausführungsbeispiels eines erfindungsgemässen Schaftinstrumentes;
- Figur 9:: Seitenansicht eines Schaftinstrumentes gemäss Figur 8, bereichsweise aufgeschnitten;
- Figur 10:: Seitenansicht eines Schaftinstrumentes gemäss den Figuren 8 und 9 in zerlegtem Zustand;
- Figur 11:: Perspektivische Darstellung eines Schaftinstrumentes gemäss den Figuren 8 bis 10 in zerlegtem Zustand.

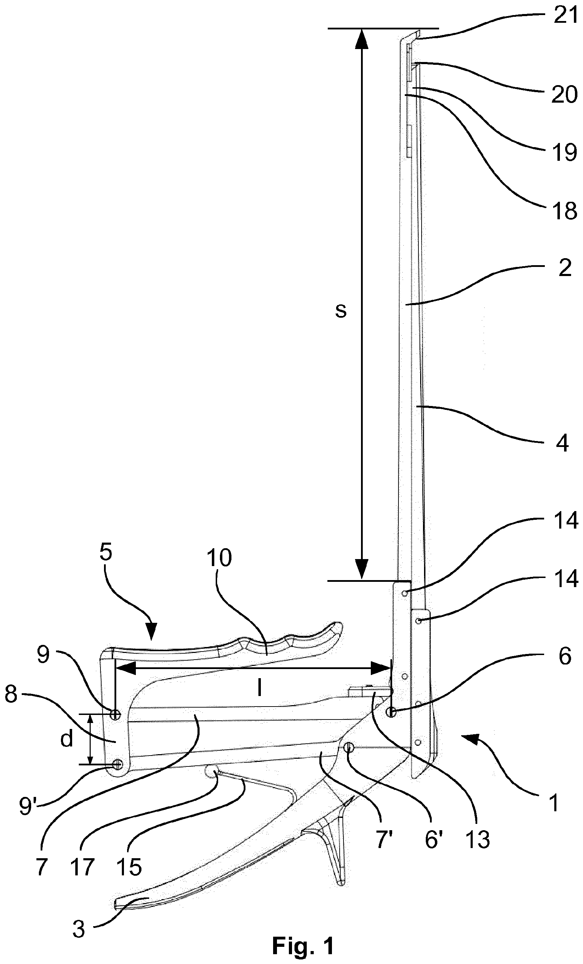

Figur 1 zeigt ein Erfindungsgemässes Schaftinstrument 1 in geöffnetem Zustand in Seitenansicht. Das besagte Instrument 1 weist ein Schaftstück 2 auf, welches am Schaftgriff 3 verankert ist. Des Weiteren ist ein Gleitstück (bzw. ein Schieber) 4 parallel zum Schaftstück 2 angeordnet. Im vorliegenden Fall handelt es sich beim Instrument 1 um eine Laminektomiestanze, bei welcher am werkzeugseitigen Ende 18 des Schaftstückes 2 ein Stanzwiderlager 21 und am werkzeugseitigen Ende 19 des Schiebers 4 ein Stanzabschnitt 20 angeordnet ist. Art und Konfiguration des Werkzeuges können jedoch je nach Anwendungszweck variieren und es wäre beispielsweise auch denkbar, dass über dem Schieber 4 ein scherenartiges Schneidewerkzeug betätigt wird.

Ferner ist zu erkennen, dass am Schaftgriff 3 die Hebelarme 7 und 7' über die Schaftgelenke 6 und 6' angelenkt sind. An ihren dem Schaftgriff 3 gegenüberliegenden Enden sind die Hebelarme 7 und 7' über die Koppelgelenke 9 und 9' mit dem Koppelabschnitt 8 des Handgriffes 5 verbunden. Der Handgriff 5 weist ferner zu dessen Betätigung einen Griffabschnitt 10 auf.

Beim gezeigten Schaftinstrument weist das Schaftstück 2 die Länge s auf. Die Länge des längeren Hebelarms 7 ist durch die Strecke 1 und der Abstand zwischen den beiden Koppelgelenken 9 und 9' in Ruhestellung durch die Distanz d gegeben.

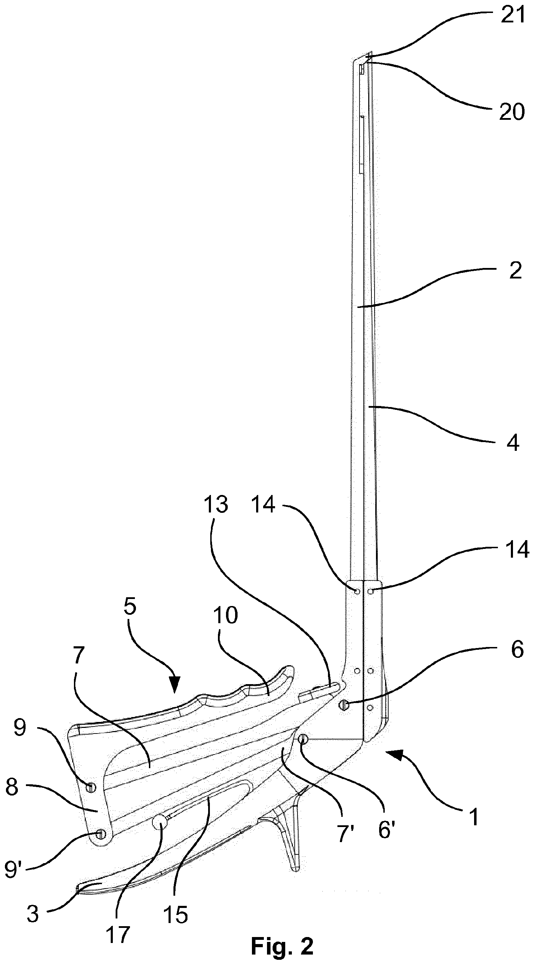

Figur 2 zeigt das erfindungsgemässe Schaftinstrument gemäss Figur 1 in geschlossenem Zustand. Es ist zu erkennen, dass durch Betätigen des Handgriffs 5 der Stanzabschnitt 20 gegen das Stanzwiderlager 21 gedrückt wird. Im Zuge dieser Bewegung wird der Schieber 4 gegen die Vorspannung der Blattfeder 15 relativ zum Schaftstück 2 in Längsrichtung verschoben.

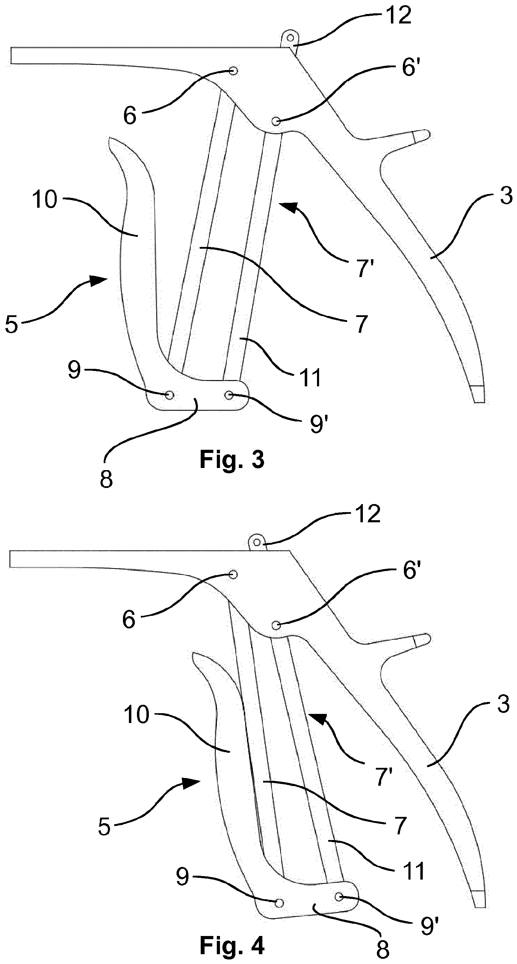

In Figur 3 ist die Griffskonfiguration eines erfindungsgemässen Schaftinstrumentes 1 in geöffnetem Zustand detailliert dargestellt. Es ist zu erkennen, dass der Hebelarm 7' als Doppelhebelarm mit einem Kraftarm 11 und einem Lastarm 12 ausgebildet ist. Das Ende des Lastarmes 12 ist dabei so ausgelegt, dass es auf das Gleitstück 4 wirken kann.

In Figur 4 ist die Griffskonfiguration gemäss Figur 3 in geschlossenem Zustand gezeigt. Beim abgebildeten Ausführungsbeispiel führt die Bewegung des Hebelarmes 7 vom geöffneten Zustand zum geschlossenen Zustand zu einer Rotation um einen Winkel von 25°. Hingegen führt dieselbe Bewegung lediglich zu einer Rotation des Handgriffes 5 um 10°.

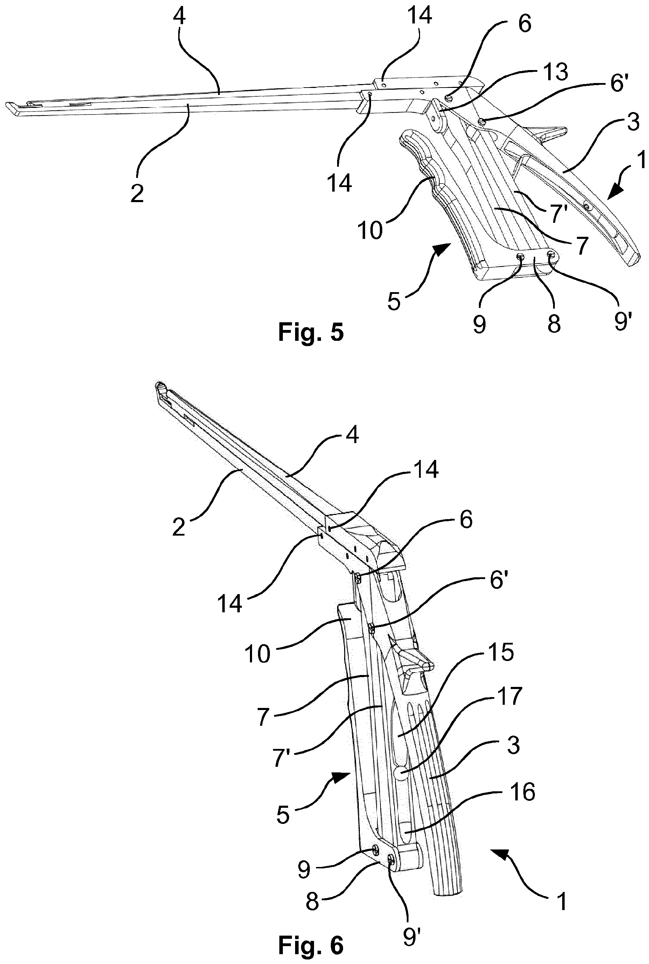

Figur 5 zeigt eine perspektivische Darstellung eines erfindungsgemässen Schaftinstrumentes gemäss den Figuren 1 und 2. Es ist zu erkennen, dass am Hebelarm 7 ein Anschlagselement 13 angebracht ist, welches die Ruhestellung des Schaftinstrumentes 1 definiert. Beim dargestellten Schaftinstrument sind das Schaftstück 2 und das Gleitstück 4 jeweils über einen reversiblen Formschluss 14, namentlich über einen Schwalbenschwanz mit Sicherungsstift, lösbar mit dem Schaftgriff 3 verbunden. Darüber hinaus ist das Federelement zum Erzeugen einer Vorspannung auf den Handgriff 5 als Blattfeder 15 ausgeführt.

Figur 6 zeigt eine weitere perspektivische Darstellung eines erfindungsgemässen Schaftinstrumentes 1 gemäss den Figuren 1 und 2. Dabei ist der Vorspannmechanismus deutlicher gezeigt. Insbesondere ist am Hebelarm 7' eine Führungsnut 16 für die Blattfeder 15 zu erkennen. An der Blattfeder 15 ist eine Kugel 17 als Gleitkörper angebracht, welcher in der Führungsnut 16 läuft.

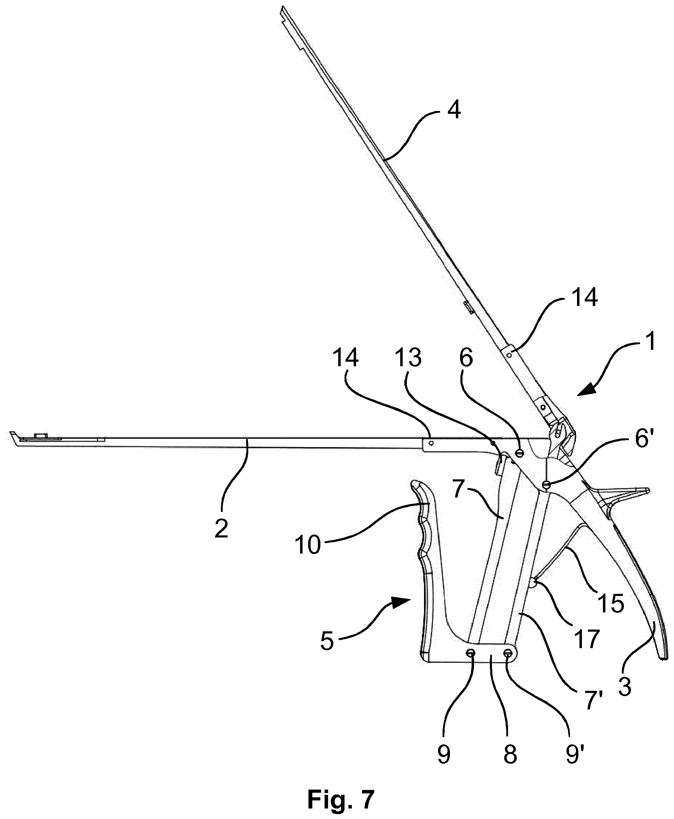

In Figur 7 ist ein erfindungsgemässes Schaftinstrument gemäss Figur 6 in einer Seitenansicht dargestellt. Dabei ist das Gleitstück bzw. der Schieber 4 vom Schaftstück 2 weggeklappt, um die beiden Teile, bspw. zu Reinigungszwecken, voneinander freizustellen. So kann das Instrument 1 in dieser Stellung in einen Sterilisierkorb zu anderen Instrumenten gelegt werden, wobei es stets zu einer Einheit verbunden bleibt.

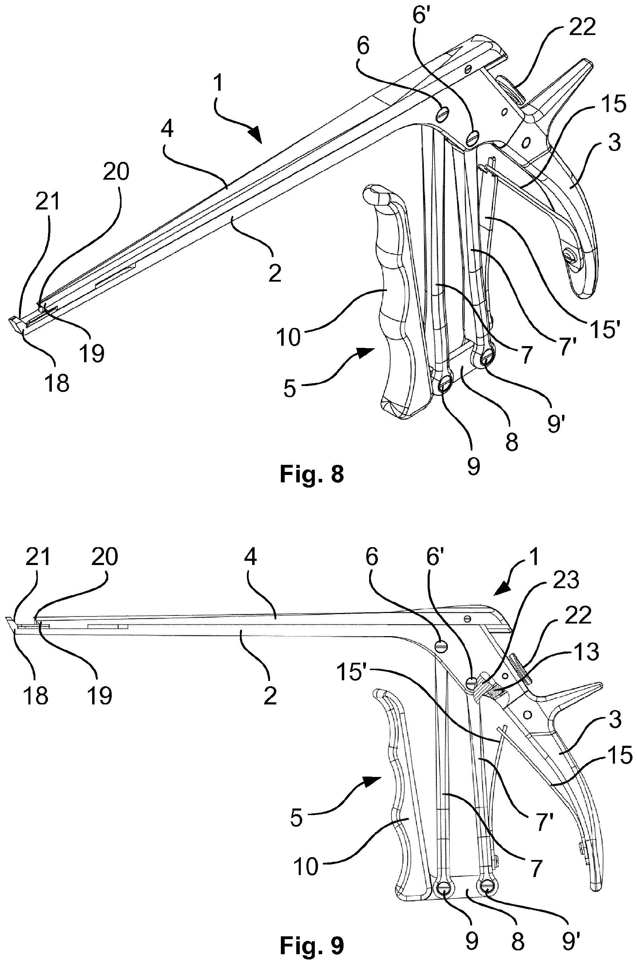

Die Figuren 8 bis 11 zeigen ein weiteres Ausführungsbeispiel eines erfindungsgemässen Schaftinstrumentes 1. Das besagte Instrument 1 weist an der bei Verwendung dem Benutzer zugewandten Seite einen Knopf 22 auf, durch dessen Betätigung das Schaftinstrument 1 zerlegt werden kann. Der Knopf 22 ist mit dem Anschlagselement 13 verbunden und unter Federvorspannung gelagert. In zusammengesetztem Zustand definiert das Anschlagselement 13 gemeinsam mit dem Anschlag 23 am Hebelarm 7' dessen Winkelstellung in der Ruhestellung.

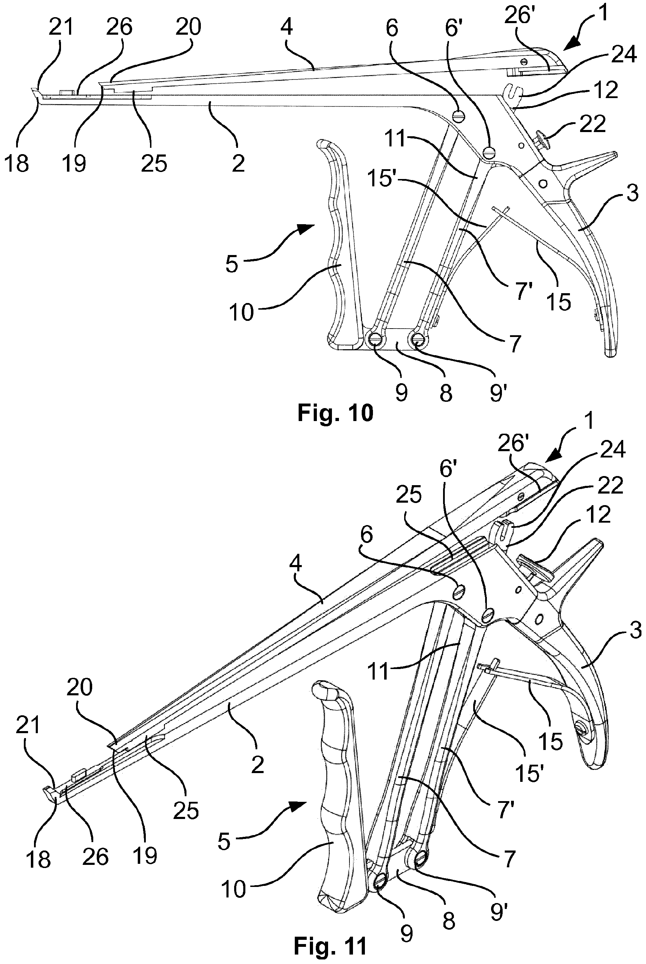

Zum Zerlegen des Schaftinstrumentes 1 muss der Benutzer den Handgriff 5 und den Griffabschnitt 10 zumindest leicht zusammendrücken, damit sich der Anschlag 23 vom Anschlagselement 13 abhebt. Danach kann der Knopf 22 gegen die Federvorspannung angehoben und um 90° gedreht werden, wodurch das Anschlagselement 13 vom Anschlag 23 entfernt und in dieser Position arretiert wird. Werden der Handgriff 5 und der Griffabschnitt 10 darauf freigegeben, bewegt sich der Hebelarm 7' entgegen der Betätigungsrichtung über die Ruhestellung hinaus. Dadurch werden die Eingriffselemente 26 und 26' am Schieber 4 bzw. am Schaftstück 2 aus den Führungsprofilen 25 und 25' ausgefahren, wodurch der Schieber 4 vollständig vom Schaftstück 2 entfernt werden kann (vgl. Fig. 10 und 11). Die Montage des Schaftinstrumentes 1 erfolgt in umgekehrter Reihenfolge.

Wie aus den Figuren 10 und 11 ersichtlich ist, ist das dem Kraftarm 11 gegenüberliegende Ende des Lastarms 12 als Gabelprofil 24 ausgeführt. Dadurch kann der Schieber 4 beim Zerlegen auf besonders einfache Weise vom restlichen Schaftinstrument getrennt werden. Das Gabelprofil 24 ist asymmetrisch ausgestaltet. Die vom Schieber 4 beaufschlagte Seite des Gabelprofils 24 weist eine höhere Materialstärke auf. Dadurch kann einer Deformation des Gabelprofils 24 durch die bei Verwendung des Schaftinstrumentes 1 auftretenden Kräfte vermieden werden.

## Patentansprüche

1. Schaftinstrument (1) für chirurgische Zwecke, mit einem Schaftstück (2), das an einem Ende fest mit einem Schaftgriff (3) verbunden ist, und einem Gleitstück (4), das relativ zum Schaftstück (2) in dessen Längsrichtung verschiebbar gelagert ist und an einem Ende derart mit einem unter Vorspannung stehenden Handgriff (5) in Wirkverbindung steht, dass durch Betätigen des Handgriffs (5) das Gleitstück (4) gegen die Vorspannung verschiebbar ist, wobei das Gleitstück (4) als parallel und neben dem Schaftstück (2) angeordneter Schieber (4) ausgebildet ist, **dadurch gekennzeichnet, dass** an wenigstens zwei voneinander beabstandeten Schaftgelenken (6, 6') am Schaftstück (2) oder am Schaftgriff (3) jeweils ein Hebelarm (7, 7') angelenkt ist und dass der Handgriff (5) einen Koppelabschnitt (8) aufweist, welcher die wenigstens zwei Hebelarme (7, 7') an voneinander beabstandeten Koppelgelenken (9, 9') gleichsinnig schwenkbar miteinander verbindet, sowie einen Griffabschnitt (10), welcher sich quer zum Koppelabschnitt (8) und zum Schaftstück (2) erstreckt.

2. Schaftinstrument (1) nach Anspruch 1, **dadurch gekennzeichnet, dass** die Koppelgelenke (9, 9') an den dem Schaftstück (2) oder Schaftgriff (3) gegenüberliegenden Enden der Hebelarme (7, 7') liegen.

3. Schaftinstrument (1) nach einem der Ansprüche 1 oder 2, **dadurch gekennzeichnet, dass** zumindest ein Hebelarm (7') als Doppelhebelarm mit einem Kraftarm (11) und einem Lastarm (12) ausgebildet ist, wobei der Kraftarm (11) mit dem Koppelabschnitt (8) des Handgriffes (5) und der Lastarm (12) mit dem Gleitstück (4) in Wirkverbindung steht.

4. Schaftinstrument (1) nach einem der Ansprüche 1 bis 3, **dadurch gekennzeichnet, dass** die Schaftgelenke (6, 6') in Querrichtung zum Schaftstück (2) versetzt angeordnet sind.

5. Schaftinstrument (1) nach einem der Ansprüche 1 bis 4, **dadurch gekennzeichnet, dass** das Verhältnis Länge (l) des längsten Hebelarms (7) zum längsten Abstand (d) zwischen zwei Koppelgelenken (9, 9') in Ruhestellung in einem Bereich von 2 bis 20, vorzugsweise von 3 bis 10, bevorzugterweise von 4 bis 6 liegt.

6. Schaftinstrument (1) nach einem der Ansprüche 1 bis 5, **dadurch gekennzeichnet, dass** an zumindest einem der Hebelarme (7, 7') ein Anschlagselement (13) angebracht ist, welches die Winkelstellung des Hebelarms (7, 7') in Ruhestellung definiert.

7. Schaftinstrument (1) nach einem der Ansprüche 1 bis 6, **dadurch gekennzeichnet, dass** der Schieber (4) zumindest teilweise lösbar mit dem Schaftstück (2) verbunden ist.

8. Schaftinstrument (1) nach Anspruch 7, **dadurch gekennzeichnet, dass** der Schieber (4) derart am Schaftstück (2) angelenkt ist, dass er über eine Klappbewegung vom Schaftstück (2) freistellbar ist.

9. Schaftinstrument (1) nach einem der vorhergehenden Ansprüche, **dadurch gekennzeichnet, dass** das Verhältnis der Länge (s) des Schaftes zur Länge (l) des längsten Hebelarms (7) in einem Bereich von 1 bis 10, vorzugsweise von 1.2 bis 4, bevorzugterweise von 1.5 bis 2.8 liegt.

10. Schaftinstrument (1) nach einem der vorhergehenden Ansprüche, **dadurch gekennzeichnet, dass** die Vorspannung durch ein am Schaftgriff (3) angebrachtes und auf einen der Hebelarme (7, 7') wirkendes Federelement, insbesondere durch eine Blattfeder (15, 15), erzeugt wird.

11. Schaftinstrument (1) nach einem der Ansprüche 1 bis 10, **dadurch gekennzeichnet, dass** das Schaftstück (2) ein Führungsprofil (25) und der Schieber (4) ein darin eingreifendes Eingriffselement (26') aufweist und/oder dass der Schieber (4) ein Führungsprofil (26) und das Schaftstück (2) ein darin eingreifendes Eingriffselement (26) ausweist.

12. Schaftinstrument (1) nach den Ansprüchen 6 und 7, **dadurch gekennzeichnet, dass** das Anschlagselement (13) in einer Verriegelungsposition die Winkelstellung des Hebelarms (7, 7') in Ruhestellung definiert und zusätzlich in eine Entriegelungsposition bewegbar, insbesondere verschiebbar oder schwenkbar, ist, wobei mit dem Anschlagselement (13) in Entriegelungsposition der Hebelarm (7, 7') entgegen der Betätigungsrichtung über die Ruhestellung hinaus bewegbar und der Schieber (4) dadurch zumindest teilweise vom Schaftstück (2) lösbar ist.

13. Schaftinstrument (1) nach den Ansprüchen 11 und 12, **dadurch gekennzeichnet, dass** der Schieber (4) durch Ausfahren von zumindest einem Eingriffselement (26, 26') aus einem Führungsprofil (25, 25') entgegen der Betätigungsrichtung vom Schaftstück (2) lösbar ist.

14. Schaftinstrument (1) nach Anspruch 3, **dadurch gekennzeichnet, dass** der Lastarm (12) am dem Kraftarm (11) gegenüberliegenden Ende ein Gabelprofil (24) aufweist, in das ein am Gleitstück (4) angebrachter Bolzen einsetzbar ist.

15. Schaftinstrument (1) nach Anspruch 14, **dadurch gekennzeichnet, dass** das Gabelprofil (24) asymmetrisch ausgestaltet ist, wobei die bei Betätigung des Schaftinstrumentes (1) von dem am Gleitstück (4) angebrachten Bolzen beaufschlagte Seite des Gabelprofils (24) eine höhere Materialstärke aufweist.

## Claims

1. Shaft-type instrument (1) for surgical purposes, with a shaft part (2), which is fixedly connected at one end to a shaft grip (3), and with a sliding part (4), which is mounted so as to be displaceable relative to the shaft part (2) in the longitudinal direction thereof and is operatively connected at one end to a pretensioned handgrip (5) in such a way that, by actuation of the handgrip (5), the sliding part (4) is displaceable counter to the pretensioning, the sliding part (4) being designed as a slide (4) arranged parallel to and next to the shaft part (2), **characterized in that** a respective lever arm (7, 7') is articulated on the shaft part (2) or on the shaft grip (3) at at least two shaft joints (6, 6') spaced apart from each other, and **in that** the handgrip (5) has a coupling portion (8) which connects the at least two lever arms (7, 7') to each other at spaced apart coupling joints (9, 9') so as to be pivotable in the same direction, and a grip portion (10) which extends transversely with respect to the coupling portion (8) and to the shaft part (2).

2. Shaft-type instrument (1) according to Claim 1, **characterized in that** the coupling joints (9, 9') lie at those ends of the lever arms (7, 7') directed away from the shaft part (2) or shaft grip (3).

3. Shaft-type instrument (1) according to either of Claims 1 and 2, **characterized in that** at least one lever arm (7') is designed as a double lever arm with a force arm (11) and a load arm (12), the force arm (11) being operatively connected to the coupling portion (8) of the handgrip (5), and the load arm (12) being operatively connected to the sliding part (4).

4. Shaft-type instrument (1) according to one of Claims 1 to 3, **characterized in that** the shaft joints (6, 6') are offset in the transverse direction with respect to the shaft part (2).

5. Shaft-type instrument (1) according to one of Claims 1 to 4, **characterized in that** the ratio of the length (1) of the longest lever arm (7) to the longest distance (d) between two coupling joints (9, 9') in the rest position is in a range of 2 to 20, preferably 3 to 10, more preferably 4 to 6.

6. Shaft-type instrument (1) according to one of Claims 1 to 5, **characterized in that** a stop element (13) is mounted on at least one of the lever arms (7, 7') and defines the angle position of the lever arm (7, 7') in the rest position.

7. Shaft-type instrument (1) according to one of Claims 1 to 6, **characterized in that** the slide (4) is connected at least partially releasably to the shaft part (2).

8. Shaft-type instrument (1) according to Claim 7, **characterized in that** the slide (4) is articulated on the shaft part (2) in such a way that it can be freed from the shaft part (2) by a swivel movement.

9. Shaft-type instrument (1) according to one of the preceding claims, **characterized in that** the ratio of the length (s) of the shaft to the length (1) of the longest lever arm (7) is in a range of 1 to 10, preferably 1.2 to 4, more preferably 1.5 to 2.8.

10. Shaft-type instrument (1) according to one of the preceding claims, **characterized in that** the pretensioning is generated by a spring element mounted on the shaft grip (3) and acting on one of the lever arms (7, 7'), in particular by a leaf spring (15, 15').

11. Shaft-type instrument (1) according to one of Claims 1 to 10, **characterized in that** the shaft part (2) has a guide profile (25') and the slide (4) has an engagement element (26') engaging therein, and/or **in that** the slide (4) has a guide profile (26) and the shaft part (2) has an engagement element (26) engaging therein.

12. Shaft-type instrument (1) according to Claims 6 and 7, **characterized in that** the stop element (13), in a locking position, defines the angle position of the lever arm (7, 7') in the rest position and is additionally movable, in particular displaceable or pivotable, to an unlocking position, wherein, with the stop element (13) in the unlocking position, the lever arm (7, 7') is movable beyond the rest position counter to the direction of actuation, and the slide (4) is thereby at least partially releasable from the shaft part (2).

13. Shaft-type instrument (1) according to Claims 11 and 12, **characterized in that** the slide (4) is releasable from the shaft part (2) by withdrawal of at least one engagement element (26, 26') from a guide profile (25, 25') counter to the direction of actuation.

14. Shaft-type instrument (1) according to Claim 3, **characterized in that** the load arm (12) has, at the end opposite the force arm (11), a fork profile (24) into which a bolt mounted on the sliding part (4) can be inserted.

15. Shaft-type instrument (1) according to Claim 14, **characterized in that** the fork profile (24) has an asymmetrical configuration, wherein the side of the fork profile (24) acted on by the bolt mounted on the sliding part (4), upon actuation of the shaft-type instrument (1), has a greater material thickness.

## Revendications

1. Instrument à tige (1) à usage chirurgical, comprenant un élément tige (2) qui est connecté fixement par une extrémité à une poignée de tige (3), et un élément coulissant (4) qui est supporté de manière déplaçable dans sa direction longitudinale par rapport à l'élément tige (2) et qui est en liaison fonctionnelle, à une extrémité, avec une poignée (5) précontrainte de telle sorte que par actionnement de la poignée (5), l'élément coulissant (4) puisse être déplacé à l'encontre de la précontrainte, l'élément coulissant (4) étant réalisé sous forme de coulisseau (4) disposé parallèlement et à côté de l'élément tige (2), **caractérisé en ce qu'**un bras de levier (7, 7') est à chaque fois articulé au niveau d'au moins deux articulations de tige espacées l'une de l'autre (6, 6') à l'élément tige (2) ou à la poignée de tige (3) et **en ce que** la poignée (5) présente une portion d'accouplement (8) qui relie l'un à l'autre de manière pivotante dans le même sens les au moins deux bras de levier (7, 7') au niveau d'articulations d'accouplement espacées l'une de l'autre (9, 9'), et une portion de poignée (10) qui s'étend transversalement à la portion d'accouplement (8) et à l'élément tige (2).

2. Instrument à tige (1) selon la revendication 1, **caractérisé en ce que** les articulations d'accouplement (9, 9') sont situées au niveau des extrémités des bras de levier (7, 7') opposées à l'élément tige (2) ou à la poignée de tige (3).

3. Instrument à tige (1) selon l'une quelconque des revendications 1 et 2, **caractérisé en ce qu'**au moins un bras de levier (7') est réalisé sous forme de double bras de levier avec un bras de force (11) et un bras de charge (12), le bras de force (11) étant en liaison fonctionnelle avec la portion d'accouplement (8) de la poignée (5) et le bras de charge (12) étant en liaison fonctionnelle avec l'élément coulissant (4).

4. Instrument à tige (1) selon l'une quelconque des revendications 1 à 3, **caractérisé en ce que** les articulations de tige (6, 6') sont disposées de manière décalée dans la direction transversale par rapport à l'élément tige (2).

5. Instrument à tige (1) selon l'une quelconque des revendications 1 à 4, **caractérisé en ce que** le rapport de la longueur (l) du bras de levier le plus long (7) à la distance la plus longue (d) entre deux articulations d'accouplement (9, 9') dans la position de repos est compris dans une plage de 2 à 20, de préférence de 3 à 10, plus préférablement de 4 à 6.

6. Instrument à tige (1) selon l'une quelconque des revendications 1 à 5, **caractérisé en ce qu'**au niveau d'au moins l'un des bras de levier (7, 7') est monté un élément de butée (13) qui définit dans la position de repos la position angulaire du bras de levier (7, 7').

7. Instrument à tige (1) selon l'une quelconque des revendications 1 à 6, **caractérisé en ce que** le coulisseau (4) est connecté au moins en partie de manière détachable à l'élément tige (2).

8. Instrument à tige (1) selon la revendication 7, **caractérisé en ce que** le coulisseau (4) est articulé à l'élément tige (2) de manière à pouvoir être libéré par un mouvement de rabattement de l'élément tige (2).

9. Instrument à tige (1) selon l'une quelconque des revendications précédentes, **caractérisé en ce que** le rapport de la longueur (s) de la tige à la longueur (l) du bras de levier le plus long (7) est compris dans une plage de 1 à 10, de préférence de 1,2 à 4, plus préférablement de 1,5 à 2,8.

10. Instrument à tige (1) selon l'une quelconque des revendications précédentes, **caractérisé en ce que** la précontrainte est générée par un élément de ressort monté sur la poignée de tige (3) et agissant sur l'un des bras de levier (7, 7'), en particulier par un ressort à lame (15, 15').

11. Instrument à tige (1) selon l'une quelconque des revendications 1 à 10, **caractérisé en ce que** l'élément tige (2) présente un profil de guidage (25') et le coulisseau (4) présente un élément d'engagement (26') s'engageant dans celui-ci et/ou **en ce que** le coulisseau (4) présente un profil de guidage (26) et l'élément tige (2) présente un élément d'engagement (26) s'engageant dans celui-ci.

12. Instrument à tige (1) selon les revendications 6 et 7, **caractérisé en ce que** l'élément de butée (13), dans une position de verrouillage, définit la position angulaire du bras de levier (7, 7') dans la position de repos et peut en outre être déplacé dans une position de déverrouillage, en particulier par coulissement ou pivotement, le bras de levier (7, 7') avec l'élément de butée (13) dans la position de déverrouillage, pouvant être déplacé à l'encontre de la direction d'actionnement au-delà de la position de repos et le coulisseau (4) pouvant de ce fait être libéré au moins en partie de l'élément tige (2).

13. Instrument à tige (1) selon les revendications 11 et 12, **caractérisé en ce que** le coulisseau (4) peut être libéré de l'élément tige (2) par sortie d'au moins un élément d'engagement (26, 26') hors d'un profil de guidage (25, 25') à l'encontre de la direction d'actionnement.

14. Instrument à tige (1) selon la revendication 3, **caractérisé en ce que** le bras de charge (12) présente, à l'extrémité opposée aux bras de force (11), un profil fourchu (24) dans lequel peut être inséré un boulon monté sur l'élément coulissant (4).

15. Instrument à tige (1) selon la revendication 14, **caractérisé en ce que** le profil fourchu (24) est réalisé sous forme asymétrique, le côté du profil fourchu (24) sollicité lors de l'actionnement de l'instrument à tige (1) par le boulon monté sur l'élément coulissant (4) présentant une plus grande épaisseur de matériau.
